# EUROPÄISCHE PATENTSCHRIFT

(11) **EP 0 644 425 B1**
(45) Veröffentlichungstag und Bekanntmachung des Hinweises auf die Patenterteilung: **30.01.2002**
(21) Anmeldenummer: 94113653.3
(22) Anmeldetag: 01.09.1994
(51) Int. Cl.: G01N 35/00, G01N 33/543

(54) **Analysengerät mit einer Vorrichtung zum Abtrennen magnetischer Mikropartikel**
Analyser having a device for separating magnetic microparticles
Analyseur, comprenant un dispositif pour la séparation de microparticules magnétiques

(30) Priorität: 17.09.1993 CH 280193
(43) Veröffentlichungstag der Anmeldung: 22.03.1995
(73) Patentinhaber: F. HOFFMANN-LA ROCHE AG, 4002 Basel (CH)
(72) Erfinder: Knobel, Rolf, CH-6343 Rotkreuz (CH)
(74) Vertreter: AMMANN PATENTANWAELTE AG BERN

(56) Entgegenhaltungen:
- EP-A- 0 136 126
- EP-A- 0 317 286
- EP-A- 0 410 645
- EP-A- 0 589 636
- WO-A-92/05443
- DE-A- 3 102 029
- US-A- 4 895 650
- PATENT ABSTRACTS OF JAPAN vol. 016, no. 186 (P-1347) 7. Mai 1992 & JP-A-04 022 867 (TOSHIBA CORP) 27. Januar 1992

## Beschreibung

Die vorliegende Erfindung betrifft ein Analysengerät der durch den Oberbegriff des Patentanspruchs 1 definierten Art.

Die Vorrichtung eignet sich beispielsweise zum Einsatz in einer Waschstation eines Analysengerätes zur automatisierten DNA-Detektion oder zur Durchführung von Immunoassays, ist aber nicht auf diese Einsatzgebiete beschränkt.

Bei vielen Analysenverfahren wie z.B. auch bei der Durchführung von Immunoassays ist die Trennung von fester und flüssiger Phase mit anschliessendem Waschen der festen Phase notwendig. Zum Waschen der festen Phase kann eine bestimmte Menge an Pufferlösung in ein Reaktionsgefäss, das die feste Phase enthält, pipettiert werden. Die feste Phase wird dadurch in der Pufferlösung suspendiert. Anschliessend wird die feste und die flüssige Phase getrennt (Separation). Die flüssige Phase kann abgesaugt werden (Aspiration ). Ein neuer Waschvorgang kann beginnen. Es werden meist mehrere Waschzyklen durchgeführt, die jeweils einen Suspensions-, Separations- und Aspirationsvorgang umfassen.

Bei Verwendung magnetischer Mikropartikel als feste Phase ist die Trennung durch Permanentmagneten im Prinzip bekannt. Die Permanentmagneten ziehen die Mikropartikel an die Wand des Reaktionsgefässes und halten sie dort fest.

Eine Vorrichtung der Eingangs erwähnten Art ist aus der EP-A-0 410 645 bekannt.

In der europäischen Patentanmeldung EP 0 136 126 wird eine Vorrichtung zur Durchführung von Trennungen während FestphasenImmunoassays beschrieben, wobei das untere Ende eines Reaktionsgefäss, welches die magnetischen Partikel enthält, zwischen zwei Permanentmagneten angeordnet ist. Die Magnetisierungsachsen liegen senkrecht zur Wand des Reaktionsgefässes. So wird eine Minimierung von magnetischen Streufeldern erreicht.

In der internationalen Anmeldung WO 92/05443 wird eine Trennvorrichtung für magnetische Mikropartikel beschrieben, wobei die Reaktionsgefässe reihenweise angeordnet sind. Ebenfalls in Reihen sind Magnetblöcke angeordnet, sodass die Reaktionsgefässe an gegenüberliegenden Seiten von Permanentmagneten umgeben sind. Die Polachsen der Magnetblöcke verlaufen parallel. Das bedingt eine Trennung durch Anhäufen der Partikel an einer Seite des Reaktionsgefässes.

In der deutschen Offenlegungsschrift DE 31 02 029 wird eine Vorrichtung zum Abtrennen von ferromagnetischen Partikeln aus Suspensionen mit Hilfe von Permanentmagneten beschrieben, wobei der Permanentmagnet seitlich angeordnet ist. Der Magnet befindet sich nur auf einer Seite des Reaktionsgefässes. Dabei ist der Magnet der Form des Reaktionsgefässes angepasst und bildet einen spitzen Winkel mit der Längsachse des Reaktionsgefässes. Die Magnetisierungsachse liegt senkrecht zur Wand des Reaktionsgefässes.

In der amerikanischen Patentschrift US 4,895,650 ist eine Trenneinrichtung beschrieben, wobei die Trennung von Mikropartikeln ebenfalls durch Permanentmagneten erreicht wird. Der Magnet befindet sich nur auf einer Seite des Reaktionsgefässes. In dieser Patentschrift wird die Beziehung zwischen Füllhöhe der Testlösung im Reagenzglas und der Position des Magneten angesprochen. Die Position des Magneten, insbesondere seine Höhe, muss mit der Füllhöhe der Testlösung im Reaktionsgefäss übereinstimmen und wird mit Hilfe von Füllmaterial im unteren Teil der Vorrichtung, die den Magneten trägt, auf die gewünschte Höhe gebracht.

Bei der Durchführung von Immunoassays ist die Füllhöhe im Reaktionsgefäss nach der Zugabe der erforderlichen Reagenzien nicht notwendigerweise einheitlich. So kann z. B. die Füllhöhe im Reaktionsgefäss nach Zugabe von Conjugatlösung geringer sein als die Füllhöhe, die nach Zugabe von Wasch-Pufferlösung entsteht. Bei dem aus US-PS 4,895,650 bekannten Analysenverfahren werden diese unterschiedlichen Füllhöhen nicht berücksichtigt.

Eine Vorrichtung zur Trennung magnetischer, als feste Phase verwendeten Partikel ist ferner in der nach dem Prioritätsdatum dieser Anmeldung veröffentlichten, älteren europäischen Patentanmeldung EP-A-0 589 636 beschrieben, deren Inhalt als Stand der Technik unter Artikel 54(3) und (4) EPÜ zu berücksichtigen ist. Die in dieser Anmeldung beschriebene Anordnung von Magneten und Leiter des magnetischen Flusses ist so eingerichtet, dass sie nicht auf einen länglichen unteren Teil des Reaktionsgefässes wirkt, sondern nur auf dem Bodenbereich des Reaktionsgefässes. Dabei wird das von den Magneten erzeugte magnetische Feld nicht unmittelbar zum Innenraum des Reaktionsgefässes geführt, sondern durch separate Leiter des magnetischen Flusses, welche zwischen den Magneten und dem Reaktionsgefäss angeordnet sind.

Die bekannten Vorrichtungen zum Trennen von magnetischen Mikropartikeln haben den Nachteil, dass ein relativ langes Zeitintervall erforderlich ist, bis alle Mikropartikel von der flüssigen Phase abgetrennt sind. Speziell bei grösseren Volumen nimmt die Separationszeit beträchtlich zu.

Eine Vorrichtung zur raschen Trennung der magnetischen Mikropartikel wird in der europäischen Patentanmeldung EP- 317286 beschrieben. Bei dieser Vorrichtung wird das Reaktionsgefäss von 4 Permanentmagneten (Magnet 1,2,3 und 4) umgeben, die gleichmässig um das Reaktionsgefäss angeordnet sind. Die Richtung des Magnetfeldes von Magnet 1 und 3 ist um 180 ° gedreht in bezug auf die Richtung des Magnetfeldes von Magnet 2 und 4.

Diese Vorrichtung hat den Nachteil, dass relativ viele Permanentmagnete eingesetzt werden müssen, um die gewünschte Beschleunigung der Trennung zu erreichen. Auch sind dadurch viele Möglichkeiten der Küvettenbewegung nicht realisierbar.

Der Erfindung liegt daher die Aufgabe zugrunde, ein Analysengerät mit einer Vorrichtung zum Abtrennen magnetischer Mikropartikel bereitzustellen, das bei unterschiedlichen Füllhöhen des Reaktionsgefässes eine rasche Trennung der in Suspension befindlichen magnetischen Mikropartikel erlaubt. Der Erfindung liegt ebenso die Aufgabe zugrunde, eine focussierte Ablagerung der magnetischen Mikropartikel zu ermöglichen.

Erfindungsgemäss wird die Aufgabe durch ein Analysengerät gemäss Patentanspruch 1 gelöst.

Bevorzugte Ausführungsformen des erfindungsgemässen Analysengeräts sind durch die abhängigen Ansprüche 2-10 definiert.

Vorzugsweise bilden die Polachsen der Magneten und die Längsachse des Reaktionsgefässes einen Winkel von 45 Grad.

Die Magneten können mit einem Magnethalter verbunden sein, der eine Nut besitzt, in die der Boden des Reaktionsgefässes hineinragt.

Die Magneten können in bezug auf das eine Suspension enthaltende Reaktionsgefäss, so angeordnet sein, dass die Polachsen der Magnete sich im Bereich der Suspensionsoberfläche oder im Bereich des Bodens des Reaktionsgefässes schneiden. Die Suspensionsoberfläche ist die Füllhöhe im Reaktionsgefäss nach Zugabe von Waschpufferlösung.

Die Aufnahmestationen zur Aufnahme der Reaktionsgefässe sind im Rahmen der vorliegenden Erfindung Aufnahmestationen einer Wascheinrichtung. Die erfindungsgemässe Kennvorrichtung lässt sich aber an allen Aufnahmestationen eines Analysengerätes einsetzen, an denen eine Trennung von fester und flüssiger Phase durchgeführt wird.

Die Wascheinrichtung besteht beispielsweise aus einem drehbaren, kreisförmigen Rotormagazin und einem Halter, der eine Resuspensions-und Aspirationsvorrichtung trägt.

In einer Wascheinrichtung ist die Anordnung von sechs Barbeitungsstationen, die gleichmässig auf einem Rotormagazin verteilt sind, vorteilhaft. Es sind jedoch auch andere Anordnungen der Bearbeitungsstationen denkbar.

Die Wascheinrichtungen können Bearbeitungsstationen zur Trennung von fester und flüssiger Phase mit Magnetanordnungen und Bearbeitungsstationen zur Resuspension ohne Magnetanordnung enthalten. Jede Magnetanordnung besteht vorteilhafterweise aus zwei Paaren von Magneten, zwischen die das Reaktionsgefäss gesetzt wird. Jede Magnetanordnung besteht also aus zwei Aufnahmestationen. Die Magneten der Aufnahmestationen können in bezug auf den Kreisdurchmesser des Rotormagazins entlang Radien angeordnet sein. Je zwei Aufnahmestationen können derart benachbart sein, dass die Radien, entlang derer die beiden Magnete angeordnet sind, einen spitzen Winkel bilden, der vorteilhafterweise 20 Grad beträgt.

Die durch die Erfindung erreichten Vorteile sind im wesentlichen darin zu sehen, dass durch die besondere Ausrichtung der Polachsen mit Schnittpunkt der Polachsen im Bereich der Suspensionsoberfläche die Magnetkraftlinien des Gesamtkraftfeldes so verlaufen, dass das magnetische Streufeld auch zur Trennung der magnetischen Mikropartikel mit ausgenutzt wird. Auch bei maximal möglicher Füllhöhe des Reaktionsgefässes sind die magnetischen Mikropartikel, die sich an der Suspensionsoberfläche befinden, im Einflussbereich des Gesamtkraftfeldes und können schnell und vollständig aus der Suspension abgetrennt werden. Nach der Abtrennung der magnetischen Mikropartikel liegt die Obergrenze der abgetrennten Mikropartikel an gegenüberliegenden Wänden des Reaktionsgefässes stets unterhalb des Flüssigkeitsspiegels, der im Verlauf der Testführung bei Zugabe von Reagenz-bzw. Waschlösungen auftritt.

Bei Ausrichtung der Polachsen mit Schnittpunkt der Polachsen im Bereich des Bodens des Reaktionsgefässes wird eine focussierte Ablagerung der magnetischen Mikropartikel an gegenüberliegenden Wänden an der Unterseite des Reaktionsgefässes ermöglicht. Diese Ausführungsform eignet sich zur Trennung von magnetischen Mikropartikeln mit hohem magnetischen Anteilen. Sie ermöglicht eine effizientere Mischung mit nachner zugegebener Flüssigkeit.

In dem erfindungsgmässen Analysengerät kann eine möglichst hohe Leistung, d.h. eine möglichst hohe Anzahl bearbeiteter Proben pro Zeiteinheit bei einer Anlage bestimmter Grösse mit geringstmöglichen Mitteln und bester Zuverlässigkeit erreicht werden.

Der Trennvorgang bzw. Separationsvorgang in einer Waschstation geht als Zeitfaktor in den Bearbeitungsprozess mit ein. Dieser Zeitfaktor wird erfindungsgemäss dadurch verringert, dass
a) die Aufnahmestationen zwei in bezug auf das Reaktionsgefäss diametral gegenüberliegende Permanentmagneten mit besonders ausgerichteter Polachse enthalten,
b) mehrere Reaktionsgefässe gleichzeitig einem Trennvorgang unterzogen werden,
c) die Reaktionsgefässe schrittweise den Resuspensions-bzw. Aspirationsprozessen zugeführt werden.

Die Taktzeiten sämtlicher Bearbeitungsstationen sind derart aufeinander abgestimmt, dass keine Totzeiten entstehen.

Ein Ausführungsbeispiel der Erfindung wird im folgenden anhand der beiliegenden Zeichnungen beschrieben. Es zeigen:
Fig. 1 und 8 einen Schnitt durch eine Aufnahmestation mit einer Trennvorrichtung mit erfindungsgemässer Magnetanordnung,
Fig. 2 und 9 ein Reaktionsgefäss im Einfluss eines magnetischen Kraftfeldes,
Fig. 3 radiale Anordnung der Bearbeitungsstationen auf einem kreisförmigen Rotormagazin,
Fig. 4 eine Gesamtansicht eines Rotormagazin,
Fig. 5 einen Schnitt durch eine Trenneinrichtung gemäss der Linie V-V in Fig. 3,
Fig. 6 Uebersichtsdarstellung der Wascheinrichtung,
Fig. 7 eine perspektivische Gesamtdarstellung eines Analysengerätes,

Beispielhaft wird ein Analysengerät zur automatisierten Durchführung eines Festphasenimmunoassays vorgestellt, bei dem die feste Phase aus magnetischen Mikropartikeln besteht und die Trennung von fester und flüssiger Phase mit Hilfe von Permanentmagneten erfolgt. Nach der Trennung sind die Mikropartikel an zwei diametral gegenüberliegenden Wandungsbereichen des Reaktionsgefässes niedergeschlagen.

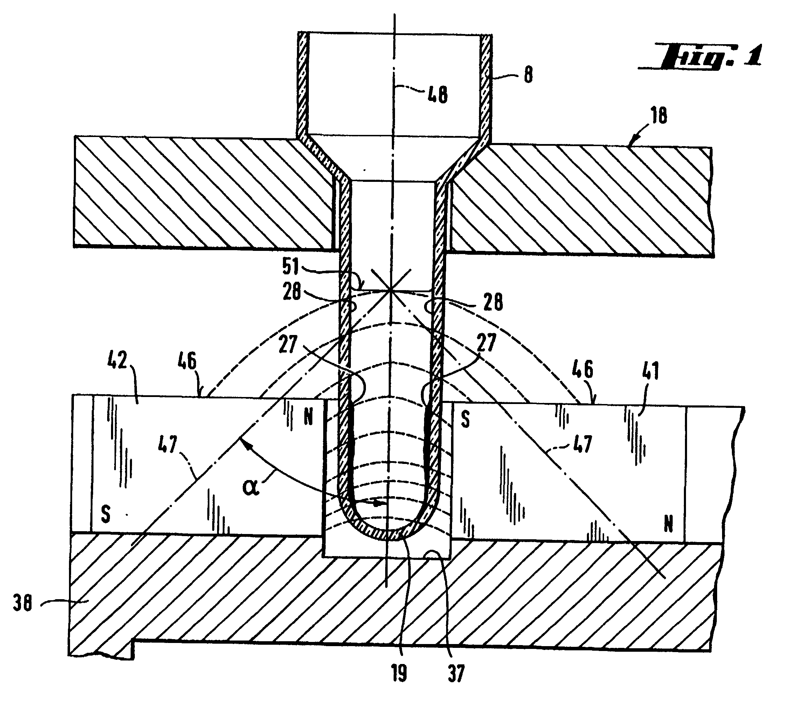

Fig. 1 zeigt eine Aufnahmestation zur Aufnahme eines Reaktionsgefässes, das eine Suspension magnetischer Mikropartikel enthält, wobei die Aufnahmestation eine Trennvorrichtung zum Abtrennen magnetischer Mikropartikel enthält und wobei die Trennvorrichtung aus zwei Magneten besteht, zwischen denen sich das Reaktionsgefäss befindet, dadurch gekennzeichnet, dass sich die Magneten 41 und 42 in bezug auf das Reaktionsgefäss 8 diametral gegenüberliegen und die Polachsen 47 der Magneten 41 und 42 und die Längsachse 48 des Reaktionsgefässes 8 einen spitzen Winkel (α) bilden. Der Winkel (α) beträgt 45 Grad.

Die Magneten 41 und 42 sind mit einem Magnethalter 38 verbunden, der eine Nut 37 besitzt.

Die Magneten 41 und 42 und der Magnethalter 38 sind Teile einer Aufnahmestation, die sich in einem Rotormagazin 18 befindet.

Die Oberkante 46 der Magnete 41 und 42 liegt unterhalb der Suspensionsoberfläche 51, an der sich die Polachsen 47 der Magnete 41 und 42 schneiden. Die magnetischen Mikropartikel 27 werden an zwei sich diametral gegenüberliegenden Wandungsbereichen 28 im Inneren der Reaktionsgefässe 8, welche den Magneten 41, 42 am nächsten stehen, abgelagert. Die Ablagerungen erfolgen in einem örtlich begrenzten Bereich, was sicherstellt, dass bei unterschiedlichen Füllhöhen des Reaktionsgefässes im Verlauf der Testdurchführung der Flüssigkeitsspiegel stets oberhalb der Ablagerungen 27 liegt und dadurch eine Resuspension zuverlässig durchgeführt werden kann.

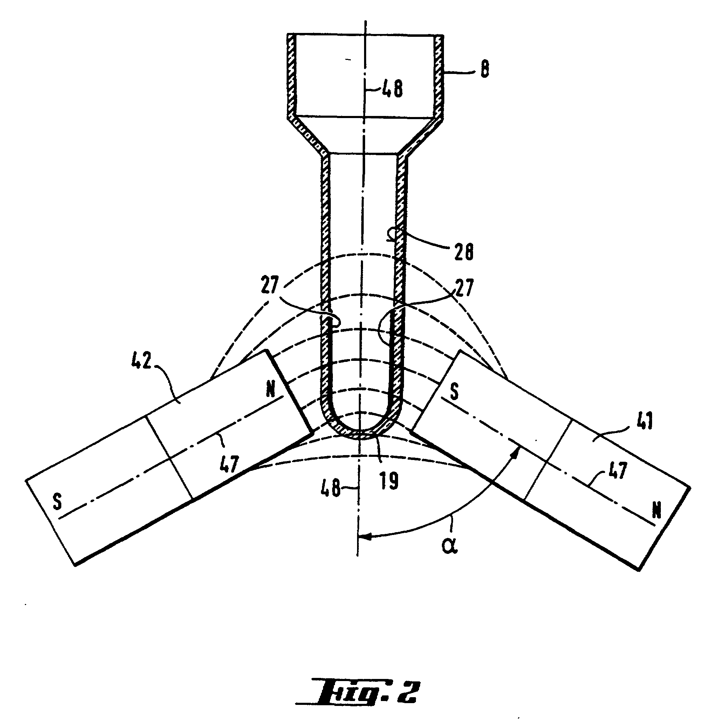

Fig. 2 zeigt die Ablagerungen der magnetischen Mikropartikel 27 an der Innenwand 28 des Reaktionsgefässes 8 im Einfluss eines magnetischen Feldes bei einer weiteren erfindungsgemässen Anordnung der Magnete 41 und 42. Die Feldlinien der Einzelmagnete sind schematisch dargestellt. Der Winkel (α), den die Polachse 47 mit der Längsachse 48 des Reaktionsgefässes 8 bildet, beträgt 45 Grad.

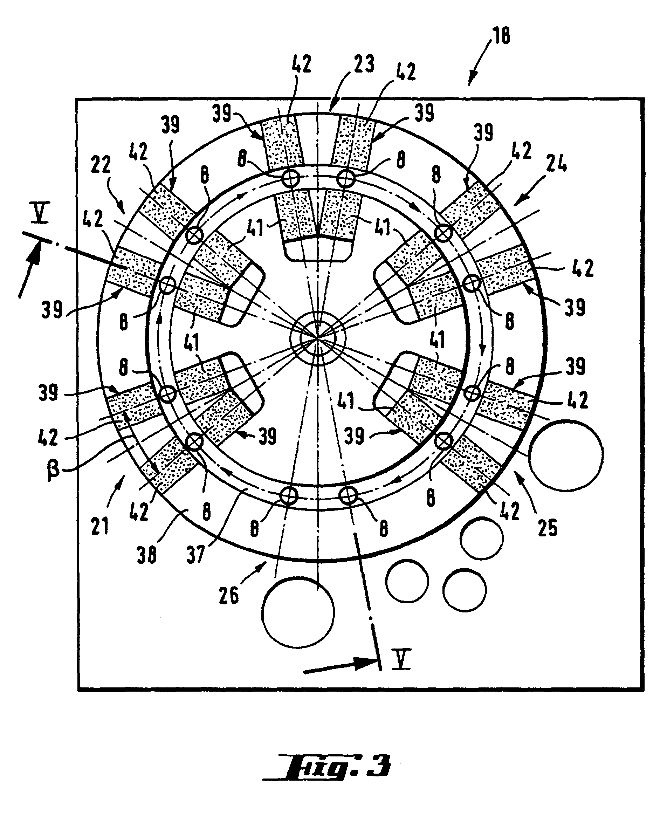

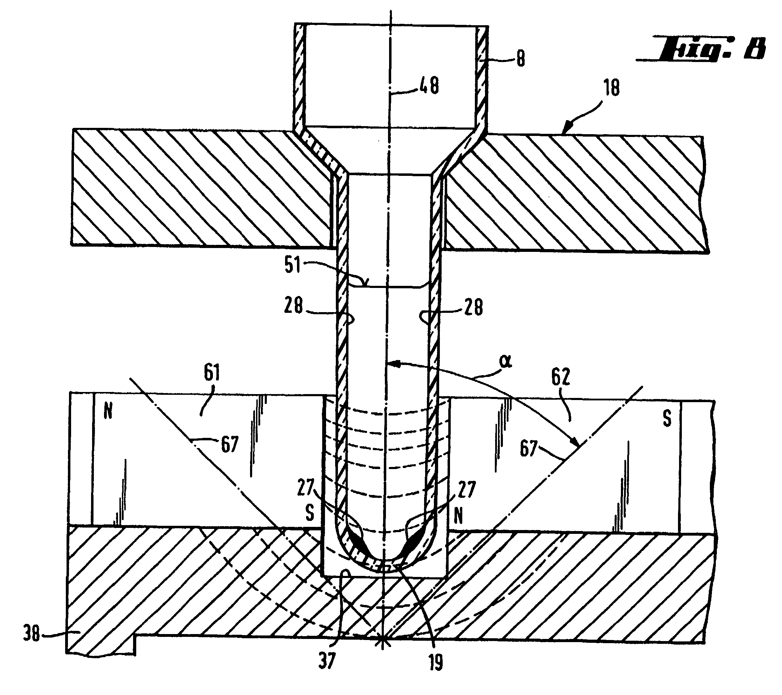

Fig.3 zeigt sechs Bearbeitungsstationen 21-26 radial auf einem kreisförmigen, drehbaren Rotormagazin 18 angeordnet, wobei die Bearbeitungsstationen 21-24 zur Trennung von fester und flüssiger Phase dienen, die Aspiration in Bearbeitungsstation 25 und die Resuspension in Bearbeitungsstation 26 erfolgt, und wobei die Bearbeitungsstationen 21-25 Vorrichtungen zum Abtrennen der magnetischen Mikropartikel enthalten. Die Bearbeitungsstationen 21-25 enthalten eine Magnetanordnung 39 bestehend aus je zwei Paaren von Magneten 41 und 42, wobei sowohl die Ausführungsform nach Fig. 1 mit den Magneten 41 und 42 als auch die Ausführungsform nach Fig. 8 mit den Magneten 61 und 62 möglich ist. Die Magneten 41 und 42 sind in bezug auf den Kreisdurchmesser des Rotormagazins 18 entlang Radien angeordnet, die einen spitzen Winkel (β) bilden. Der Winkel (β) beträgt 20 Grad. Zwischen den Magneten 41 und 42 befinden sich die Reaktionsgefässe 8 in der Nut 37 des Magnethalters 38. Die Magnetanordnungen 39 sind jeweils im Winkel von 60 Grad voneinander beabstandet.

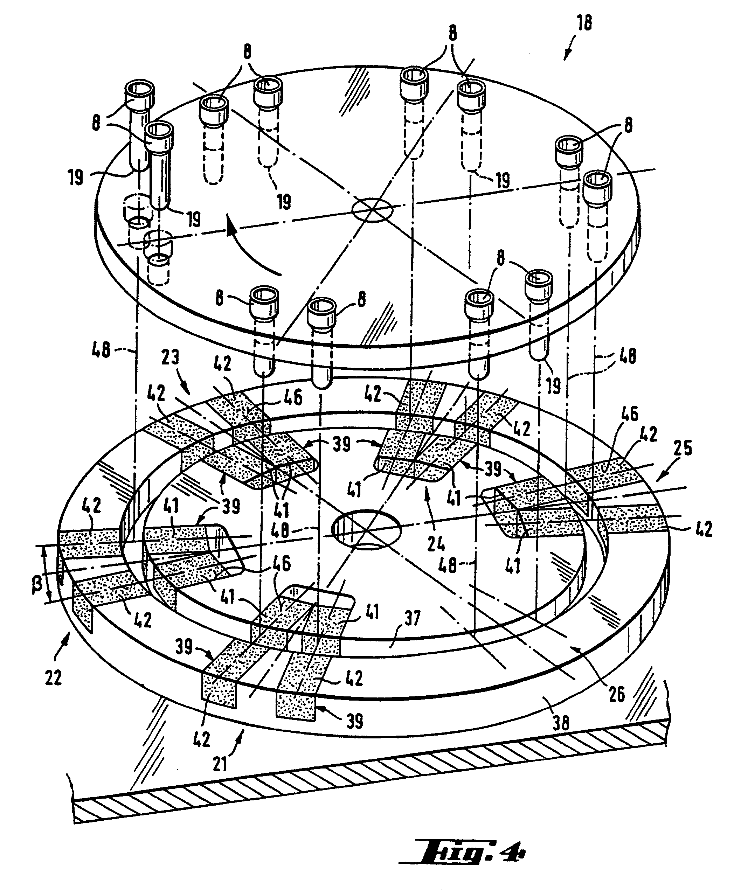

Fig. 4 zeigt eine perspektivische Gesamtansicht des Rotormagazins 18. Man erkennt die Bearbeitungsstationen 21-26. Man erkennt, dass die Längsachsen 48 der Reaktionsgefässe 8 parallel zur Rotordrehachse laufen. Der Boden 19 der Reaktionsgefässe 8 liegt über der Nut 37 des Magnethalters 38. 12 Reaktionsgefässe 8 sind in 6 Zweiergruppen im Randbereich des Rotors 18 auf einem gemeinsamen Teilkreis angeordnet. Jeder Zweiergruppe ist jeweils im Winkel von 60 Grad voneinander beabstandet.

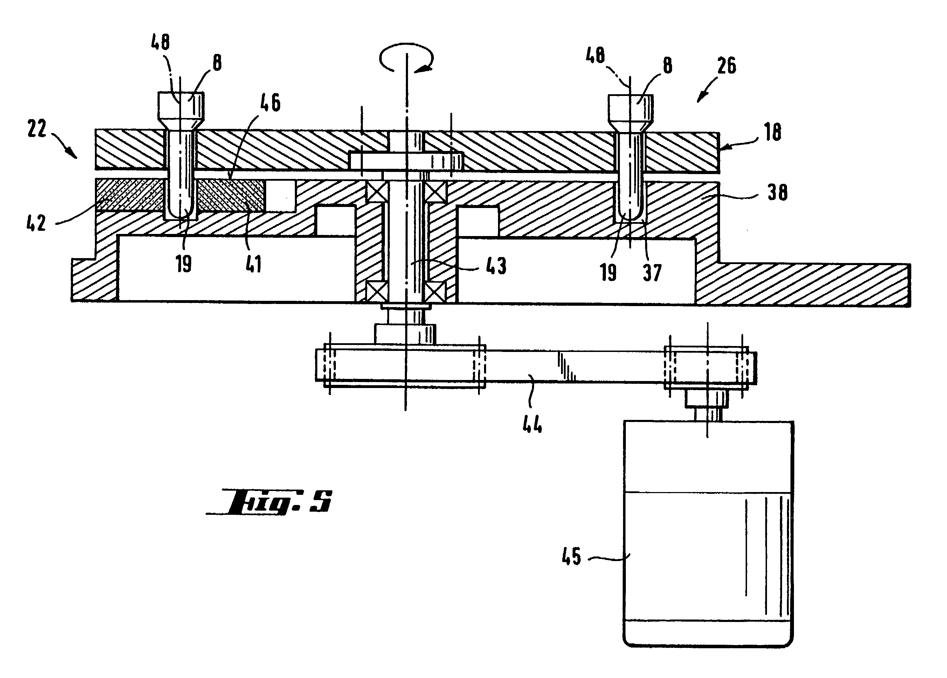

Fig. 5 zeigt einen Schnitt durch eine Trenneinrichtung, von Bearbeitungsstation 22 nach 26 mit den Reaktionsgefässen 8. Die Längsachsen 48 der Reaktionsgefässe 8 laufen parallel zur Rotordrehachse. Bearbeitungsstation 22 enthält die Magnete 41 und 42, während Bearbeitungsstation 26 keine Magnete enthält und zur Resuspension der magnetischen Mikropartikel dient. Das Rotormagazin 18 ist über eine im Magnethalter 38 gelagerte Welle 43 und einen Zahnriemenantrieb 44 mit einem Antriebsmotor 45 verbunden.

Der Antriebsmotor 45 ist derart taktweise steuerbar, dass die Reaktionsgefässe 8 jeweils in den entsprechenden Bearbeitungsstationen zu stehen kommen, solange wie es die erforderlichen Prozesse verlangen. Das Rotormagazin dreht sich bei jedem Transporttakt um 30 Grad. Durch die stets identische Magnetanordnung in den verschiedenen Stationen werden die magnetischen Mikropartikel stets an identischen Bereichen an der Innenwand des Reaktionsgefässes gesammelt.

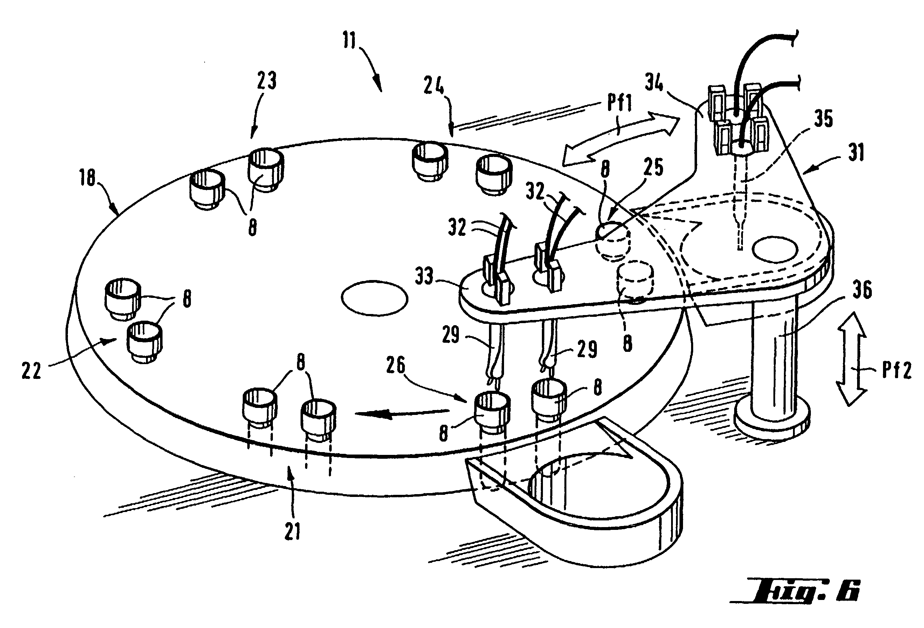

In Fig. 6 ist eine erfindungsgemässe Wascheinrichtung 11 dargestellt, die das Rotormagazin 18 sowie einen Halter 31 mit einer Resuspensions-und Aspirationsvorrichtung enthält. Man erkennt wieder die Bearbeitungsstationen 21-26.

Die in den Bearbeitungstationen 25 und 26 eingesetzten Aspirations- und Resuspensionsvorrichtungen sind in einem Halter 31 festgelegt und über Kapillarröhrchen 32 an ein Versorgungssystem (Pumpe, Vorratsbehälter usw.) angeschlossen. Der Halter 31 ist als zweiarmiger Hebel ausgebildet, wobei an dem einen Hebelende 33 die Einspritzdüsen 29 und an dessen anderem Hebelarm 34 die Aspirationskanülen 35 angeordnet sind. Der Hebelarm ist um seine Drehachse 36 verschwenkbar sowie vertikal absenkbar. Diese Verstellung ist in Fig. 6 mit den Pfeilen Pf1, Pf2 angedeutet. Die Grösse der Winkelverstellung, der Abstand der Einspritz- und Aspirationselemente und deren Anordnung an den entsprechenden Hebelenden des Halters ist dabei derart aufeinander abgestimmt, dass die Einspritzelemente 29 mit den in der Bearbeitungsstation 26 oder die Aspirationkanüle 35 mit den in den Bearbeitungsstationen 25 befindlichen Reaktionsgefässen 8 zur Bearbeitung in Flucht gebracht werden können. Die nicht in Arbeitsposition befindlichen Elemente befinden sich dabei über einem Abtropfbehälter.

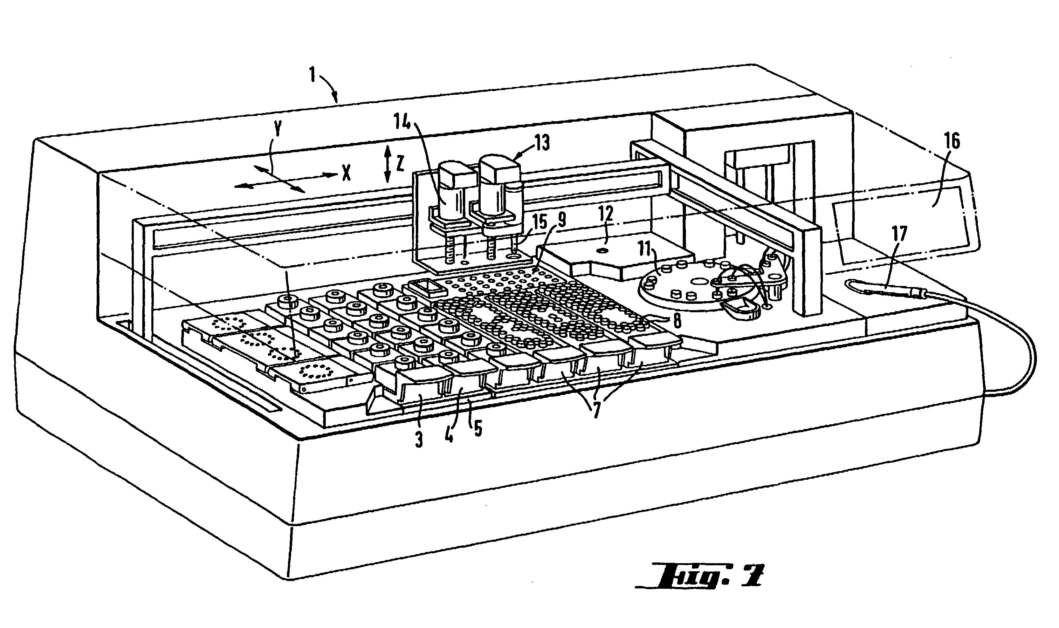

Fig. 7 zeigt eine Uebersichtsdarstellung des erfindungsgemässen Analysengerätes, welches beispielsweise zur Durchführung von DNA-Detektionen ausgelegt ist. In dem Analysengerät 1 sind enthalten:
Einrichtungen zur Durchführung obengenannter DNA-Detektionen, hier z.B. zwei Racks 3, 4 mit Reagenzien auf einem Schütteltisch 5, drei Racks 7 mit Einwegreagenzbehältern 8, ein temperierbarer Inkubator 9, eine Wascheinrichtung 11 und ein Photometer 12.

Der Proben- und Reagenzientransfer sowie der Reaktionsgefässe-Transfer wird durch einen im x-y-Koordinatensystem bewegbaren Transferkopf 13 ermöglicht, welcher eine Pipettiereinrichtung 14 sowie einen Reaktionsgefässegreifer 15, beide in z-Richtung bewegbar, aufweist.

Die Wascheinrichtung kann über den Greifer 15 der Transfereinrichtung 13 be- und entladen werden.

Ueber eine Bedienebene 16 und / oder einen Barcode-Lesegriffel 17 können Prozessparameter eingegeben werden. Die CPU steuert und koordiniert sämtliche Prozessvorgänge.

Fig. 8 zeigt eine weitere erfindungsgemässe Ausführungsform. Im Unterschied zu Fig. 1 sind die in Fig. 8 dargestellten Magneten 61 und 62 um 180° gedreht. Nord-und Südpol der in Fig. 8 dargestelleten Magneten 61 und 62 liegen sich im Bodenbereich 19 des Reaktionsgefässes 8 diametral gegenüber. Die Polachsen 67 der Magnete 61 und 62 und die Längsachse 48 des Reaktionsgefässes 8 bilden einen spitzen Winkel (α) und schneiden sich im Bereich des Bodens 19. Die Magneten 61 und 62 sind ebenso wie in Fig. 1 mit dem Magnethalter 38 verbunden, der eine Nut 37 besitzt, in die der Boden 19 des Reaktionsgefässes 8 hineinragt. Die magnetischen Mikropartikel 27 werden an zwei sich diametral gegenüberliegenden Wandungsbereichen 28 im Inneren der Reaktionsgefässe 8 im Bereich des Bodens 19 focussiert. Die Magneten 61 und 62 und der Magnethalter 38 sind Teile einer Aufnahmestation, die sich in einem Rotormagazin 18 befindet.

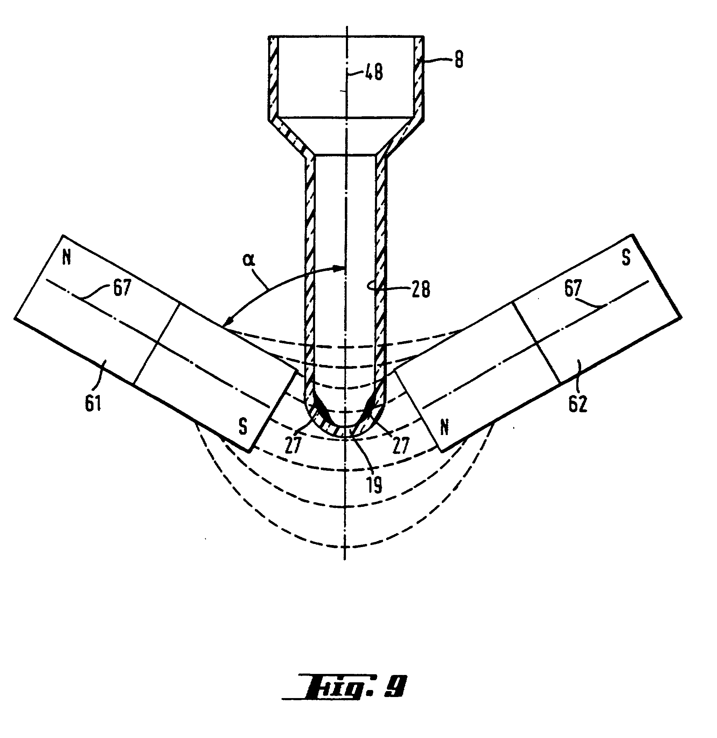

Fig. 9 zeigt die Ablagerungen der magnetischen Mikropartikel 27 an der Innenwand 28 des Reaktionsgefässes 8 im Einfluss eines magnetischen Feldes bei einer weiteren erfindungsgemässen Anordnung der Magnete 61 und 62. Die Feldlinien der Einzelmagnete sind schematisch dargestellt. Der Winkel (α), den die Polachse 67 mit der Längsachse 48 des Reaktionsgefässes 8 bildet, beträgt 45 Grad.

Die zwei oben beschriebenen Magnetanordnungen, d. h. die Anordnung gemäss Fig. 1 und die Anordnung gemäss Fig. 8 können auch kombiniert verwendet werden. Die speziellen Magnete 41, 42, 61, 62 haben den Vorteil, dass sie für beide Magnetanordnungen verwendet werden können.

## Patentansprüche

1. Analysengerät, welches
ein Reaktionsgefäss (8) und wenigstens eine Bearbeitungsstation zur Aufnahme eines Reaktionsgefässes umfasst,
wobei das Reaktionsgefäss (8) einen länglichen unteren Teil mit im wesentlichen konstanter Querschnitt, einen geschlossenen Boden (19), und eine Längsachse (48) hat, und eine Suspension magnetischer Mikropartikel (27) enthält, welche Suspension eine Oberfläche (51) hat,
wobei die wenigstens eine Bearbeitungsstation eine Trennvorrichtung zum Abtrennen der magnetischen Mikropartikel enthält,
wobei die Trennvorrichtung aus zwei Magneten (41, 42 bzw. 61, 62) besteht, und ein länglicher Bereich des unteren Teils des Reaktionsgefässes (8) zwischen dem Nordpol des einen Magneten (41, 61) und dem Südpol des anderen Magneten (42, 62) angeordnet ist, wobei der Nordpol des einen Magneten (41, 61) gegenüber dem Südpol des anderen Magneten (42, 62) liegt, und
wobei sich zwischen jedem der Magneten und dem Reaktionsgefäss ausschliesslich ein Luftspalt befindet,
welches Analysengerät **dadurch gekennzeichnet ist, dass**
die Polachse (47) jedes der Magneten (41, 42) mit der Längsachse (48) des Reaktionsgefässes (8) einen spitzen Winkel α bildet.

2. Analysengerät gemäss Anspruch 1, **dadurch gekennzeichnet, dass** der spitze Winkel α 45 Grad beträgt.

3. Analysengerät gemäss Anspruch 1, **dadurch gekennzeichnet, dass** die Magneten (61, 62) mit einem Magnethalter (38) verbunden sind, der eine Nut (37) besitzt, in die der Boden (19) des Reaktionsgefässes (8) hineinragt.

4. Analysengerät gemäss Anspruch 1, **dadurch gekennzeichnet, dass** die Magneten (41, 42), in bezug auf das Reaktionsgefäss (8) so angeordnet sind, dass die Oberkante (46) der Magnete (41, 42) unterhalb der Suspensionsoberfläche (51) liegt und dass sich die Polachsen (47) der Magnete (41, 42) an der Suspensionsoberfläche (51) schneiden.

5. Analysengerät gemäss Anspruch 1, **dadurch gekennzeichnet, dass** die Magneten (61, 62) in bezug auf das Reaktionsgefäss (8) so angeordnet sind, dass die Pole beider Magnete (61, 62) auf einer Höhe angeordnet sind, die im Bereich des Bodens (19) liegt und dass sich die Polachsen (67) der Magnete (61) und (62) in einem Punkt schneiden, der entlang der Längsachse des Reaktionsgefässes und auf einer Höhe im Bereich des Bodens (19) liegt.

6. Analysengerät gemäss Anspruch 1, **dadurch gekennzeichnet, dass** n Bearbeitungsstationen zur Aufnahme der Reaktionsgefässe auf einem kreisförmigen Rotormagazin (18) angeordnet sind, wobei n eine ganze Zahl zwischen 1 und 20 ist.

7. Analysengerät gemäss Anspruch 6, **dadurch gekennzeichnet, dass** das Rotormagazin (18) Teil einer Wascheinrichtung (11) ist, welche mindestens eine Bearbeitungsstation (25) zur Aspiration, mindestens eine Bearbeitungsstation (26) zur Resuspension von magnetischen Partikeln, und mehrere Bearbeitungsstationen (21-24) zur Trennung von fester und flüssiger Phase enthält, wobei jede der Bearbeitungsstationen zur Trennung (21-24) und zur Aspiration (25) eine Aufnahmestation mit einer Trennvorrichtung gemäss Anspruch 4 oder 5 zum Abtrennen der magnetischen Mikropartikel enthält.

8. Analysengerät gemäss Anspruch 6 und 7, **dadurch gekennzeichnet, dass** die Aufnahmestationen in den Bearbeitungsstationen (21-25) eine paarweise Anordnung (39) von Magneten (41, 42 bzw. 61, 62) enthalten, wobei die Magneten (41, 42 bzw. 61, 62) in bezug auf den Kreisdurchmesser des Rotormagazins (18) entlang Radien angeordnet sind, die einen spitzen Winkel β bilden.

9. Analysengerät gemäss Anspruch 8, **dadurch gekennzeichnet, dass** der Winkel β 20 Grad beträgt.

10. Analysengerät gemäss einem der Ansprüche 6-9, **dadurch gekennzeichnet, dass** das Rotormagazin (18) über eine im Magnethalter (38) gelagerte Welle (43) und einen Zahnriemenantrieb (44) mit einem Antriebsmotor (45) verbunden ist, der derart taktweise steuerbar ist, dass das Reaktionsgefäss (8) jeweils in den Bearbeitungsstationen (21-26) zu stehen kommt.

## Claims

1. An analytical device comprising a reaction vessel (8) and at least one treatment station for receiving a reaction vessel, the reaction vessel, (8) comprising an elongate bottom part with a subtanstially constant cross-section, a closed base (19) and a longitudinal axis (48), and containing a suspension of magnetic microparticles (27), which suspension has a surface (51), the at least one treatment station containing a separating device for separating the magnetic microparticles, the separating device consisting of two magnets (41, 42; 61, 62) and an elongate zone of the bottom part of the reaction vessel (8) being disposed between the north pole of one magnet (41, 61) and the south pole of the other magnet (42, 62), the north pole of one magnet (41, 61) being situated opposite the south pole of the other magnet (42, 62) and solely one air gap is situated between each of the magnets and the reaction vessel, which analytical device is **characterised in that** the pole axis (47) of each of the magnets (41, 42) forms an acute angle α with the longitudinal axis (48) of the reaction vessel (8).

2. An analytical device according to claim 1, **characterised in that** the acute angle α is 45°.

3. An analytical device according to claim 1, **characterised in that** the magnets (61, 62) are connected to a magnet holder (38) which has a groove (37) into which the base (19) of the reaction vessel (8) projects.

4. An analytical device according to claim 1, **characterised in that** the magnets (41, 42) are so disposed with respect to the reaction vessel (8) that the top edge (46) of the magnets (41, 42) lies beneath the suspension surface (51) and the pole axes (47) of the magnets (41, 42) intersect at the suspension surface (51).

5. An analytical device according to claim 1, **characterised in that** the magnets (61, 62) are so disposed with respect to the reaction vessel (8) that the poles of the two magnets (61, 62) are situated at a level lying in the region of the base (19) and that the pole axes (67) of the magnets (61, 62) intercept at a point situated along the longitudinal axis of the reaction vessel and at a height in the region of the base (19).

6. An analytical device according to claim 1, **characterised in that** n treatment stations for receiving the reaction vessels are disposed on a circular rotor magazine (18), n being an integer between 1 and 20.

7. An analytical device according to claim 6, **characterised in that** the rotor magazine (18) is part of a washing device (11) which contains at least one treatment station (25) for aspiration, at least one treatment station (26) for the resuspension of magnetic particles and a plurality of treatment stations (21-24) for separating solid and liquid phases, each of the treatment stations for separation (21-24) and for aspiration (25) containing a receiving station with a separating device according to claim 4 or 5 for separating the magnetic microparticles.

8. An analytical device according to claims 6 and 7, **characterised in that** the receiving stations in the treatment stations (21-25) contain a paired arrangement (39) of magnets (41, 42; 61, 62), the magnets (41, 42; 61, 62) being disposed, with respect to the diameter of the circle of the rotor magazine (18) along radii which form an acute angle β.

9. An analytical device according to claim 8, **characterised in that** the angle β is 20°.

10. An analytical device according to any one of claims 6 to 9, **characterised in that** the rotor magazine (18) is connected, via a shaft (43) mounted in the magnet holder (38) and a toothed belt drive (44), to a drive motor (45) which is cyclically controllable in such manner that the reaction vessel (8) in each case comes to rest in the treatment stations (21-26).

## Revendications

1. Analyseur, qui comprend
un réacteur (8) et au moins une station de travail pour recevoir un réacteur, tels que
le réacteur (8) comprend une partie inférieure allongée à section transversale sensiblement constante, un fond fermé (19), et un axe longitudinal (48), et contient une suspension de microparticules magnétiques (27), suspension qui comporte une surface (51),
la station de travail unique au moins contient un dispositif de séparation pour séparer les microparticules magnétiques,
le dispositif de séparation se compose de deux aimants (41, 42 ou 61, 62), et une zone allongée de la partie inférieure du réacteur (8) est agencée entre le pôle nord d'un premier aiment (41, 61) et le pôle sud de l'autre aimant (42, 62), le pôle nord du premier aiment (41, 61) étant tourné vers le pôle sud de l'autre aimant (42, 62) et
exclusivement un entrefer se trouvant entre chacun des aimants et le réacteur,
cet analyseur étant **caractérisé en ce que**
l'axe polaire (47) de chacun des aimants (41, 42) forme un angle aigu α avec l'axe longitudinal (48) du réacteur (8).

2. Analyseur selon la revendication 1, **caractérisé en ce que** l'angle aigu α est de 45 degrés.

3. Analyseur selon la revendication 1, **caractérisé en ce que** les aimants (61, 62) sont connectés à un support (38) d'aimants qui comporte une rainure (37) dans laquelle fait saillie le fond (19) du réacteur (8).

4. Analyseur selon la revendication 1, **caractérisé en ce que** les aimants (41, 42) sont agencés par rapport au réacteur (8) d'une manière telle que l'arête supérieure (46) des aimants (41, 42) est située au-dessous de la surface (51) de la suspension et que les axes polaires (47) des aimants (41, 42) se coupent à la surface (51) de la suspension.

5. Analyseur selon la revendication 1, **caractérisé en ce que** les aimants (61, 62) sont agencés par rapport au réacteur (8) d'une manière telle que les pôles des deux aimants (61, 62) sont agencés à une hauteur qui est dans la zone du fond (19) et que les axes polaires (67) des aimants (61) et (62) se coupent en un point qui est situé le long de l'axe longitudinal du réacteur et à une hauteur dans la zone du fond (19).

6. Analyseur selon la revendication 1, **caractérisé en ce que** n stations de travail sont agencées pour recevoir les réacteurs sur un chargeur rotatif circulaire (18), et n est un nombre entier compris entre 1 et 20.

7. Analyseur selon la revendication 6, **caractérisé en ce que** le chargeur rotatif (18) fait partie d'une installation de lavage (11) qui contient au moins une station de travail (25) d'aspiration, au moins une station de travail (26) de remise en suspension de particules magnétiques et plusieurs stations de travail (21 à 24) de séparation de phases solide et liquide, et dans lequel chacune des stations de travail de séparation (21 à 24) et d'aspiration (25) contient une station réceptrice incluant un dispositif de séparation conforme à la revendication 4 ou 5 pour séparer les microparticules magnétiques.

8. Analyseur selon la revendication 6 ou 7, **caractérisé en ce que** les stations réceptrices des stations de travail (21 à 25) contiennent un agencement (39) d'aimants (41, 42 ou 61, 62) par paires, et les aimants (41, 42 ou 61, 62) sont agencés, par rapport au diamètre du cercle du chargeur rotatif (18) le long de rayons qui forment un angle aigu β.

9. Analyseur selon la revendication 8, **caractérisé en ce que** l'angle β est de 20 degrés.

10. Analyseur selon l'une quelconque des revendications 6 à 9, **caractérisé en ce que** le chargeur rotatif (18) est connecté au moyen d'un arbre (43) logé dans le support (38) d'aimant et d'un entraînement (44) par courroie crantée à un moteur d'entraînement (45) qui peut être commandé de façon cyclique d'une manière telle que le réacteur (8) vient respectivement s'immobiliser dans les stations de travail (21 à 26).
